# EUROPEAN PATENT SPECIFICATION

(11) **EP 1 495 724 B1**
(45) Date of publication and mention of the grant of the patent: **05.11.2008**
(21) Application number: 04015356.1
(22) Date of filing: 30.06.2004
(51) Int. Cl.: A61B 17/02

(54) **Surgical retractors**
Wundhalter
Ecarteurs chirurgicaux

(30) Priority: 10.07.2003 JP 2003272999
(43) Date of publication of application: 12.01.2005
(73) Proprietor: Showa IKA Kohgyo Co., Ltd., Aichi-ken (JP)
(72) Inventor: Nohara, Yutaka, Koshigaya-shi Saitama-ken (JP); Oribe, Kazuya, Minato-ku Tokyo (JP); Takamido, Hiroshi, Nagoya-shi, Aichi-ken (JP)
(74) Representative: Grünecker, Kinkeldey, Stockmair & Schwanhäusser Anwaltssozietät

(56) References cited:
- GB-A- 920 691
- US-A- 5 363 841
- US-A- 5 908 382
- US-A- 6 042 540
- US-B1- 6 302 842
- US-B1- 6 468 207

## Description

The present invention relates to surgical retractors according to the preamble of claim 1. The present invention pertains to surgical retractors that can prevent a handle portion of the surgical retractors from being raised higher even if a deep portion of the incision needs to be held open by a blade.

Such surgical retractors are already known from US 6,302,842 B. The described surgical retractors are provided with a pair of arms, which are openable and closable via a hinge portion. The arms include a blade. A pair of handles are provided on said pair of arms respectively. A bent arm portion is provided at an extremity side of each of said arms.

US 6,042,540 A discloses a side loading surgical retractor. Said retractor comprises interchangeable retractor blades. The side loading retractor blade has a distal end that may be angled. The retractor further comprises arms, a hinge and handles.

US 5,363,841 A refers to a retractor for spinal surgery. The retractor comprises supporting arms which are connected at an angle to the retractor blades. The retractor includes at an angle to the retractor blades. The retractor includes a rack and pinion assembly which includes a bar, a first arm and a second arm.

Surgical retractors for ensuring an operative field by spreading and holding open a surgical incision have been disclosed, for example, in Japanese Publication of Registered Utility Model Applications 2,131,233. The surgical retractors disclosed in the publication are obtained by devising a so-called nasal speculum and allow the incision to be spread by inserting an extremity portion thereof into the incision and closing grippers. However, the surgical retractors have a drawback in that it is difficult to hold open the incision to a fixed extent because the grippers have to be kept closed by the user during the operation.

An ecarteur for holding a surgical incision to a fixed extent has been disclosed in Japanese Patent Application Laid-open H09-234,204. The ecarteur has a pair of branch portions disposed in parallel with each other, each of the base edge thereof being connected to each other by means of a connecting portion. With this connecting portion, each of extremity portions thereof is capable of moving to and away from each other elastically. In addition, the ecarteur has a positioning member, disposed between the pair of branch portions, for regulating the position of the pair of branch portions, thereby allowing the distance between the extremity portions thereof to be kept constant during operation.

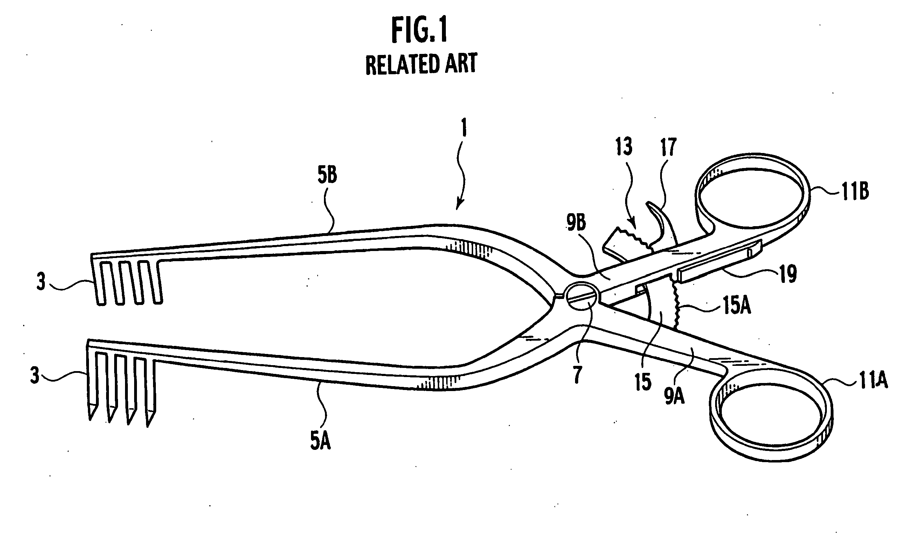

Another example of conventional surgical retractors has been described in "MIZUHO" ORTHOPEDIC IMPLANTS & INSTRUMENTS (MIZUHO Co., Ltd., December 2002). As shown in Fig.1, the surgical retractors 1 for holding open an incision that has been made, for example, over a patient's spinal cord have been comprised of a pair of arms 5A, 5B that are openably and closably connected with each other by means of a hinge portion 7, the arm 5A(5B) having at its extremity portion a blade 3 for spreading and holding the incision open. Also, the arm 5A(5B) is integrally provided with a handle 9A(9B) for performing an open/close operation of the pair of arms 5A, 5B. At the end of the handles 9A, 9B are provided respectively rings 11A, 11B for user's fingers to be inserted.

When a user inserts his or her fingers into the rings 11A, 11B and closes the handles 9A, 9B, the arms 5A, 5B pivot around the hinge 7 and the extremity portions of the arms 5A, 5B open accordingly. In order to hold the extremity portions open, the handles 9A, 9B are provided at a position adjacent to the hinge 7 with a lock mechanism 13 for locking the handles 9A, 9B against each other.

The lock mechanism 13 is comprised of a plate 15 that is formed into an arc shape around the hinge 7 and secured on one handle 9A, a ratchet finger member 17 that is pivotably provided on the other handle 9B, the end portion of the member 17 being capable of meshing with a ratchet teeth portion 15A formed on the peripheral of the plate 15, and a leaf spring 19 that is provided on the handle 9B in order to deflect the ratchet finger member 17 into engagement with the ratchet teeth portion 15A.

In case of the conventional surgical retractors 1 having such a configuration, when the blades 3 are inserted deep into an incision made on a patient body in an attempt to spread and hold open a deep portion of the incision, the arms 5A, 5B, the handles 9A, 9B, and rings 11A, 11B extrude aslant from the incision. In other words, the deeper the blades 3 are inserted into the incision, the steeper the inclination of the arms 5A, 5B, the handles 9A, 9B, and rings 11A, 11B becomes and, in a worst case, they come to stand substantially vertically from the incision.

So, there must be a problem in that the arms 5A, 5B, the handles 9A, 9B, and rings 11A, 11B tend to interfere with surgical operation.

It is an object of the present invention to provide surgical retractors which do not tend to interfere with surgical operation.

The above and other objects of the invention are achieved by surgical retractors according to claim 1.

According to the present invention, since blades for holding open a surgical incision when the incision needs to be held open are provided at an end of the bent arms that are formed at an end of the arms so as to bend downward and the bend line is parallel with the arm when seen from a lateral side, even when the blade is inserted deep beneath a skin in the incision, the bend line at the end of the blade can be maintained substantially parallel with the surface of the patient's body. Therefore, the arms and handles are prevented from being raised, thereby solving the aforementioned problem.

In the accompanying drawings:
Fig.1 is an illustration of conventional surgical retractors;
Fig.2 is a perspective illustration of surgical retractors according to the present invention;
Fig.3 is a lateral illustration of surgical retractors according to the present invention; and,
Fig.4 is an illustration of surgical retractors according to the present invention in use for a surgical incision.

Surgical retractors according to an embodiment of the present invention will be explained in detail hereinafter with reference to the accompanying drawings. In the drawings, an identical reference numeral is used to specify an identical element that demonstrates the same function as the aforementioned conventional one does, in order to avoid undue repetition in explanation.

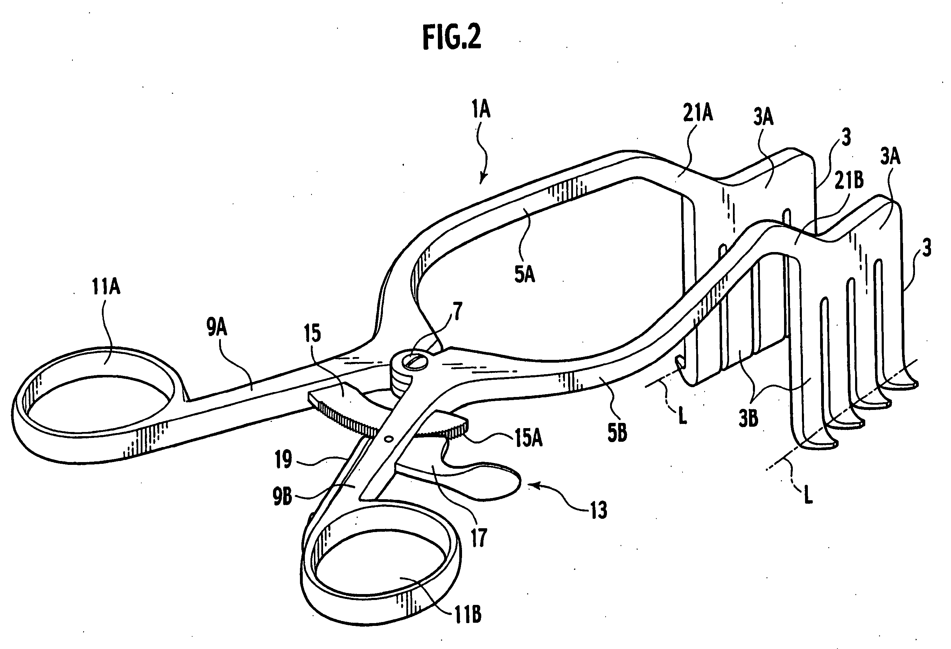

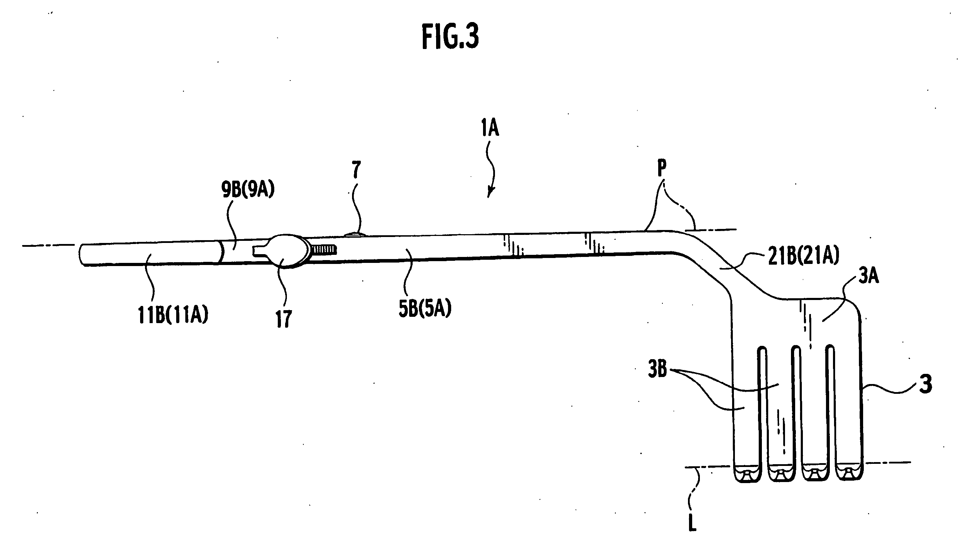

Referring to Figs. 2 and 3, surgical retractors 1A according to an embodiment of the present invention are composed of a pair of arms 5A, 5B which are connected with each other by means of a hinge portion 7 so as to open/close and provided respectively with a blade 3 for spreading and holding an incision open at an end portion of the arms 5A, 5B, and a pair of handles 9A, 9B formed on the arms 5A, 5B for performing an open/close operation of the arms 5A, 5B, as in the case of the aforementioned conventional surgical retractors 1.

Also, the surgical retractors 1A are provided with bent arms 21A, 21B that bend downward in relation to the arms 5A, 5B at the end of the arms 5A, 5B, respectively, the arms 21A, 21B having at their own ends the blades 3. The tip or lower portion of each blade 3 bends away from each other (outward) at a position of the bend line L. Seen from a lateral side, the bend line L is formed so as to be substantially parallel with the plane P formed by the arm 5A, 5B and the handles 11A, 11A, as shown in Fig.3.

More particularly, the bent arms 21A, 21B bend downward at an angle of about 45 degree in relation to the arms 5A, 5B, as shown in Fig.3. At the end of the bent arms 21A, 21B is provided a blade back portion 3A that can be supported horizontally when supporting the arms 5A, 5B horizontally and extends from the arms 5A, 5B. In other words, it is understood from Fig.3 that the blade back portion 3A is formed so as to be parallel with the arm 5A, 5B, when the blade back portion 3A and the arms 5A are seen from their lateral side.

Moreover, the blade back portion 3A is provided integrally with a plural of finger portions 3B that are formed so as to extend downward and substantially orthogonal with the blade back portion 3A. The lower end of the finger portions 3B bends outward at the bent line L.

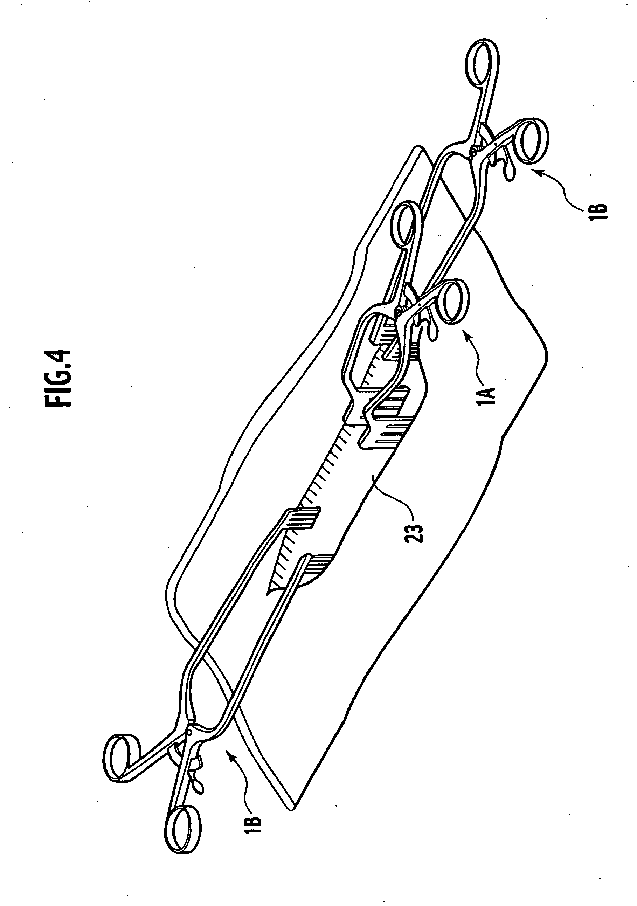

Such a configuration allows the following way of using the surgical retractors 1 when performing a surgical operation for a patient's spinal cord, for example (see Fig.4). When an incision 23 made on his or her back is spread widen, at first, the edge of the incision 23 is spread to a certain extent at a portion of the incision 23 adjacent to the end thereof using smaller sized surgical retractors 1B. Next, at the center portion of the incision 23, blades 3 of larger sized surgical retractors 1A are inserted deep into the incision 23 until the extremity portion of the blades 3 reaches beneath the skin. Then, the incision 23 is spread wide to an appropriate extent by closing the handles 9A, 9B and the arms 5A, 5B are locked with each other by the lock mechanism 13. In this situation, the arms 5A, 5B and handle 9A, 9B of the surgical retractors 1A can be overlaid partially on the smaller surgical retractors 1B. This can be realized because the bent arms 21A, 21B are bent downward from the arm 5A, 5B and also the bend line L at which the lower portions of the blades 3 are bent outward is substantially parallel with the arms 5A, 5B and handles 9A, 9B, seen from their lateral side.

In other words, even when a deep portion of the incision needs to be spread wide by inserting the blades 3 deep beneath the skin at the center portion of the incision 23, the arms 5A, 5B, the handles 9A, 9B, and the rings 11A, 11B are not raised upward higher but laid substantially horizontally, thereby preventing the arms 5A, 5B, the handles 9A, 9B and rings 11A, 11B from interfering with the surgical operation. This means that the aforementioned problem accompanied with a conventional type of surgical retractors is solved.

The surgical retractors according to the present invention are applicable in spreading and holding a surgical incision open.

## Claims

1. Surgical retractor (1A) provided with a pair of arms (5A, 5B) which are openable and closable via a hinge portion (7) and each of the arms (5A,5B) includes at a distal extremity thereof a blade (3) for spreading and holding open an incision and a pair of handles (9A, 9B) for performing an open/close operation of said pair of arms (5A, 5B), the pair of handles (9A, 9B) being provided on said pair of arms (5A, 5B) respectively, wherein downward bent arm portions (21A,21B) are provided at the distal ends of the respective arms (5A,5B), and bend lines (L) formed each at the distal end of the respective blade (3) provided at an extremity end of the bent arm portion (21A,21 B), whereby the plane in which the bend line (L) moves is parallel to the plane in which arms (5A,5B) move, the bend of the bent arm portions (21A,21B) from arms (5A,5B) being towards the plane of motion of the bend lines and each handle (9A,9B), arm (5A,5B), blade (3) unit being a single rigid unit,
**characterized in that**
each bent arm portion (21A,21B) is provided at the end thereof with a plane blade back portion (3A) and the blade back portion (3A) is opposite to the blade (3), whereby each blade (3) comprises a plurality of fingers (3B) only extending downwards from the blade back portion (3A), said fingers being bent at the extremities thereof, the bends being arranged along the bend line (L), the plane in which the blade back portion (3A) moves is substantially parallel to the plane in which arms (5A,5B) move and the arms (5A,5B) and blade back portion (3A) are offset.

## Patentansprüche

1. Chirurgischer Retraktor (1A), der mit einem Paar Schenkel (5A, 5B) versehen ist, die über einen Gelenkabschnitt (7) geöffnet und geschlossen werden können, wobei jeder der Schenkel (5A, 5B) an einem äußeren distalen Ende desselben ein Blatt (3) zum Aufspreizen und Offenhalten eines Einschnitts umfasst, sowie mit einem Paar Griffe (9A, 9B) zum Durchführen eines Vorgangs des Öffnens/Schließens der paarigen Schenkel (5A, 5B), wobei die paarigen Griffe (9A, 9B) jeweils an den paarigen Schenkeln (5A, 5B) vorhanden sind, nach unten gebogene Schenkelabschnitte (21A, 21 B) an den distalen Enden der jeweiligen Schenkel (5A, 5B) vorhanden sind und Biegelinien (L) jeweils an dem distalen Ende des jeweiligen Blattes (3) ausgebildet sind, das an einem äußeren Ende des gebogenen Schenkelabschnitts (21A, 21B) vorhanden ist, wobei die Ebene, in der sich die Biegelinie (L) bewegt, parallel zu der Ebene ist, in der sich die Schenkel (5A, 5B) bewegen, die Biegung der gebogenen Schenkelabschnitte (21A, 21B) von den Schenkeln (5A, 5B) in Richtung der Ebene der Bewegung der Biegelinien verläuft und jede Einheit aus Griff (9A, 9B), Schenkel (5A, 5B) und Blatt (3) eine einzelne starre Einheit ist,
**dadurch gekennzeichnet, dass**
jeder gebogene Schenkelabschnitt (21A, 21B) an seinem Ende mit einem planen Blatt-Rückenabschnitt (3A) versehen ist und der Blatt-Rückenabschnitt (3A) dem Blatt (3) gegenüberliegt, wobei jedes Blatt (3) eine Vielzahl von Fingern (3B) umfasst, die sich von dem Blatt-Rückenabschnitt (3A) nur nach unten erstrecken, die Finger an ihren äußeren Enden gebogen sind, die Biegungen entlang der Biegelinie (L) angeordnet sind und die Ebene, in der sich der Blatt-Rückenabschnitt (3A) bewegt, im Wesentlichen parallel zu der Ebene ist, in der sich Schenkel (5A, 5B) bewegen, und die Schenkel (5A, 5B) und der Blatt-Rückenabschnitt (3A) versetzt sind.

## Revendications

1. Ecarteur chirurgical (1A), muni d'une paire de bras (5A, 5B) qui peuvent être ouverts et fermés par l'intermédiaire d'une partie de charnière (7), et chacun des bras (5A, 5B) comprend à une extrémité distale de celui-ci une lame (3) pour écarter et maintenir ouverte une incision, et d'une paire de poignées (9A, 9B) pour effectuer une opération d'ouverture/fermeture de ladite paire de bras (5A, 5B), la paire de poignées (9A, 9B) étant prévue sur ladite paire de bras (5A, 5B), respectivement, dans lequel des parties de bras (21A, 21B) courbées vers le bas sont prévues aux extrémités distales des bras respectifs (5A, 5B), et des lignes de courbure (L) sont formées chacune à l'extrémité distale de la lame respective (3) prévue à une extrémité terminale de la partie de bras courbée (21A, 21B), de telle sorte que le plan dans lequel se déplace la ligne de courbure (L) est parallèle au plan dans lequel se déplacent les bras (5A, 5B), la courbure des parties de bras courbées (21A, 21B) à partir des bras (5A, 5B) se dirigeant vers le plan de déplacement des lignes de courbure, et chaque unité de poignée (9A, 9B), bras (5A, 5B) et lame (3) étant une unité rigide unique,
**caractérisé en ce que**
chaque partie de bras courbée (21A, 21B) est munie
à son extrémité d'une partie arrière de lame plane (3A), et la partie arrière de lame (3A) se trouve à l'opposé de la lame (3), de manière à ce que chaque lame (3) comprenne une pluralité de doigts (3B) s'étendant uniquement vers le bas à partir de la partie arrière de lame (3A), lesdits doigts étant courbés à leurs extrémités, les courbures étant agencées le long de la ligne de courbure (L), le plan dans lequel se déplace la partie arrière de lame (3A) étant substantiellement parallèle au plan dans lequel se déplacent les bras (5A, 5B), et les bras (5A, 5B) et la partie arrière de lame (3A) étant décalés.
